(19) Europäisches Patentamt / European Patent Office / Office européen des brevets

(11) **EP 4 686 486 A1**

(12) **EUROPEAN PATENT APPLICATION**

(43) Date of publication:
04.02.2026 Bulletin 2026/06

(21) Application number: 24191857.2

(22) Date of filing: 30.07.2024

(51) International Patent Classification (IPC):
**A61L 26/00** *(2006.01)* **A61K 35/14** *(2015.01)*
**A61P 17/02** *(2006.01)*

(52) Cooperative Patent Classification (CPC):
**A61L 26/0057; A61K 35/14; A61L 26/0004;
A61L 26/0009; A61L 26/0061; A61P 17/02;**
A61L 2400/04; A61L 2400/06

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Paul Hartmann AG
89522 Heidenheim (DE)**

(72) Inventor: **SMOLA, Hans
66121 Saarbrücken (DE)**

(74) Representative: **Ter Meer Steinmeister & Partner
Patentanwälte mbB
Nymphenburger Straße 4
80335 München (DE)**

(54) **GEL COMPOSITION COMPRISING WHOLE BLOOD**

(57) The present invention relates to a gel composition for applying to a wound of a patient comprising (i) whole blood, (ii) polymer, and (iii) a coagulation accelerator, wherein the coagulation accelerator is a clay mineral. The invention further relates to a method for preparing the present gel composition, to the present composition for use in wound treatment, to a kit for preparing the present composition and to a method for treating a wound.

Figure 2: PDGF-AB concentration in whole blood versus different polymers/hydrogels

**Description**

The present invention relates to a gel composition for applying to a wound of a patient comprising (i) whole blood, (ii) polymer, and (iii) a coagulation accelerator, wherein the coagulation accelerator is a clay mineral. The invention further relates to a method for preparing the present gel composition, to the present composition for use in wound treatment, to a kit for preparing the present composition and to a method for treating a wound.

**Technical Background**

A wound can be regarded as the separation of the contiguity of tissues of the skin, wherein this can be combined with loss of substance. Wounds can *inter alia* be characterized by the kind of impact, its size and the state of the healing.

The healing of wounds is based on the ability of the skin to regenerate tissue, such as epithelial tissue, connective and supporting tissue. The regeneration of tissue is a complex occurrence of cell activities overlapping each other, wherein said cell activities promote the healing process step by step. According to the literature, there are four main phases of healing. In the first phase, the hemostasis phase, the bleeding is stopped, and thrombin initiates the formation of a fibrin mesh. In the second phase, the defensive/inflammatory phase, specific white blood cells and macrophages successively enter the wound to destroy bacteria and remove debris. In addition, growth factors are provided to attract immune system cells to the wound and to facilitate tissue repair. The third phase is the proliferative phase. After the cleaning of the wound, said phase is to fill and cover the wound. During wound filling, granulation tissue fills the wound bed with connective tissue, and new blood vessels are formed. Subsequently, the wound margins contract and pull towards the wound center. In the last stage of said phase, epithelial cells are formed which cover the wound with epithelium. The fourth phase is the maturation phase. In said phase, the new tissue matures, i.e. it becomes stronger and more flexible with the reorganization of collagen fibers.

It is reported that the metabolism during wound healing is dependent on biochemical reactions which, when having properly taken place, ensure a good healing of the wound. For example, with reference to Shula VK, et al.: "Evaluation of pH measurement as a method of wound assessment", J. Wound Care, 2007; 16(7), pp. 291-294, a slightly acidic pH value of about 5.5 corresponding to the pH value of the outer surface of the skin is considered to promote oxygen perfusion, decreased bacteria presence, decreased proteolytic activity from proteases and enhanced fibroblast growth. There are several approaches to support the distinct healing phases of a wound. For example, in WO 2010/000451 and WO 2011/141454 there are wound dressings ensuring a humid environment of the wound to promote the healing process.

As indicated above, several factors are important for the healing of wounds. For example, the platelet derived growth factor (in short PDGF) appears to play a major role. Thus, there were attempts to promote the healing process by providing whole blood or components thereof such as blood serum.

EP 1 294 414 B1 relates to a composition to improve the repair and to regenerate cartilaginous tissues and its use in the manufacture of a medicament for tissue repair. Said composition *inter alia* comprises a mixture of whole blood and a polymer. Said composition, however, seems to be improvable with regard to the speed of clotting and the release of growth factors such as PGDF as well as the state of composition which is applied to the site of administration.

US 9,180,142 B2 relates to a wound dressing prepared from whole blood that is clotted *ex vivo* to form a sheet of clotted blood that is applied onto the skin over a sight of skin injury. For this, blood is withdrawn from a subject and mixed with an anticoagulant and a coagulation accelerator such as kaolin and subsequently spread onto a support matrix, where it is allowed to clot. Such a procedure appears to be improvable with regard to the adaptation of the sheet which might have to be applied to different kinds and sizes of wounds. For adapting the corresponding dressing to the wound, said dressing has to be cut to the size thereof which might result in the contamination of the dressing. With regard hereto, any contact of the operator such as a doctor, nurse with the blood dressing should be avoided due to possible infections with communicable diseases such as HIV. In addition, the procedure according to US 9,180,142 B2 might result in the binding of growth factor in the sheet/wound dressing such that they are not at the disposal for supporting the wound healing.

Hence, it is an object of the present invention to provide a composition which overcomes the shortcomings of the prior art.

In particular, a composition should be provided which is easily applicable and which enables advantageous wound healing. In particular, said composition should provide a sufficient amount of growth factors to the wound.

Furthermore, advantageous healing properties should be achieved also for wounds having a reduced cell activity and/or cell density.

Moreover, a composition for treating poorly healing wounds, such as chronic and/or ischemic wounds, should be provided.

In summary, there is still a need for a composition for treating wounds, in particular poorly healing wounds.

Hence, it was an object of the present invention to overcome at least one of the above problems.

**Summary of the invention**

Thus, the subject of the present invention is a gel composition for being applied to a wound of a patient, the composition comprising

(i) whole blood,

(ii) a polymer, and

(iii) a coagulation accelerator,

wherein the coagulation accelerator is a clay mineral.

A further aspect of the present invention is a method for preparing a composition according to the invention, comprising

(a) providing a volume of whole blood,

(b) providing a volume of a polymer comprising coagulation accelerator,

(c) mixing the volume whole blood from step (a) with the volume of polymer comprising coagulation accelerator from step (b).

Another aspect of the present invention is a composition according to the invention for use in the treatment of a chronic wound.

A further aspect of the invention is a kit for preparing the composition according to the invention comprising

- one or more blood withdrawal devices with container for the withdrawn whole blood

- one or more container(s) comprising polymer and coagulation accelerator; and

- device for mixing whole blood and the mixture of polymer and coagulation accelerator.

Finally, the invention relates to a method for treating a wound comprising the steps of

(a) providing a volume of whole blood,

(b) providing a volume of a polymer comprising coagulation accelerator,

(c) mixing the volume whole blood from step (a) with the volume of polymer comprising coagulation accelerator from step (b),

(d) applying the mixture from step (b) on the wound,

(e) optionally further applying a secondary dressing over the wound,

wherein the coagulation accelerator is a clay mineral.

**Figures**

Figure 1 shows the PDGF-AB concentration on whole blood versus the concentration of kaolin.
Figure 2 shows the PDGF-AB concentration in whole blood versus different polymers/hydrogels.

**Detailed description**

The definitions are relevant in connection with the embodiments of the present invention.
The meaning of the term "comprising" is to be interpreted as encompassing all the specifically mentioned features as well as optional, additional unspecified ones, whereas the term "consisting of" only includes those features as specified. Therefore, "comprising" includes as a limiting case the meaning of "consisting of'.

In this application the term "about" may be understood as +/- 5% of the corresponding value.

The terms "% by weight" or "% w/w" are understood to refer to the weight amount of the component relative to the total weight of the adhesive silicone layer if not specified otherwise.

The meaning of the term "alkyl" is to be interpreted to encompass linear, branched and cyclic alkyl residues. Thus, for example an "alkyl having 1 to 3 carbon atoms" encompasses methyl, ethyl, propyl, isopropyl and cyclopropyl.

The composition according to the present invention comprises (i) whole blood. Whole blood can be regarded as human blood from a standard blood draw, preferably from venous or arterial circulation. Thus, whole blood has not been modified and contains all of its ingredients such as hematocrit and plasma. Hematocrit designates the fraction of cellular blood components such as red and white blood cells, while plasma designates an 90% aqueous solution of proteins, salts and low-molecular compounds such monosaccharides.

The whole blood contained in the composition according to the present invention can be autologous or non-autologous blood, preferably autologous blood. Autologous blood refers to the blood of the person themselves to be treated with the present composition.

The composition according to the present invention comprises (ii) a polymer. A polymer can be referred to as a compound consisting of large groups of atoms that are made from combinations of smaller molecules.

It is preferred that the polymer is a synthetic-based polymer, a natural-based polymer or mixtures thereof.

Synthetic-based polymers are polymers that are synthesized or modified for example in a laboratory. In a preferred embodiment, the synthetic-based polymer has a number average weight of 2.500 to 250.000.000 g/mol, preferably 5.000 to 5.000.000 g/mol, in particular 50.000 to 1.000.000 g/mol.

Non-limiting examples of synthetic-based polymers are polyalkyleneoxide, poly(meth)acrylate, poly(eth)acrylate, polyalkyl(meth)acrylate, polyalkyl(meth)acrylate, vinyl-based polymers, caprolactam-based polymers, caprolactone-based polymers, polyurethane, polyurea and polyurethane-polyurea-copolymer.

A polyalkylene oxide is a compound which can be represented by the formula $H-(O-A)_n-OH$, wherein A is an alkylene group, preferably a linear alkylene group comprising 2 to 6 carbon atoms, in particular 2 or 3 carbon atoms. Preferred polyalkylene oxides are polyethylene oxide (also referred to as polyethylene glycol), polypropylene oxide (also referred to as polypropylene glycol) and a copolymer from polyethylene oxide and polypropylene oxide.

Poly(meth)acrylate and poly(eth)acrylate are polymers obtained by polymerisation of the corresponding acid, i.e. (meth)acrylic and eth(acrylic) acid, respectively. Polyalkyl(meth)acrylate and polyalkyl(eth)acrylate are alkylesters, preferably alkylesters with 1 to 6 carbon atoms, in particular methyl or ethyl ester of the before-mentioned (meth)acrylic and eth(acrylic) acids, respectively.

A vinyl-based polymer is a polymer derived from compounds containing a vinyl group, wherein the vinyl group can be substituted or unsubstituted, preferably substituted. Substituents can be aromatic groups such as benzene, alkyl groups, preferably alkyl groups with 1 to 6 carbon atoms, or other substituents such as halogens, hydroxy and nitrile. Particularly preferred is polyvinyl alcohol.

Caprolactam-based polymers and caprolactone-based polymers are the polymers of the corresponding caprolactam and caprolactone. Caprolactam and caprolactone can be substituted, for example, with the substituents as mentioned above, or unsubstituted, preferably unsubstituted.

Polyurethane is a polymer composed of organic units joined by urethane (carbamate) links. Polyurethane is represented by the following formula

$$[-(C=O)-NH-R-NH-(C=O)-O-R'-O-]_n,$$

wherein R and R' are aliphatic or aromatic residues.

Polyurea is a polymer represented by the following formula

$$-[-NH-R-NH-(C=O)-NH-R'-NH-(C=O)-]_n,$$

wherein R and R' are aliphatic or aromatic residues.

Polyurethane-polyurea-copolymer comprises units of each of the before mentioned formulae.

Alternatively and more preferred, the polymer can be a natural-based polymer. Natural-based polymers are polymers that have natural origins such as animals, plants, and algae. It is preferred that the polymer (ii), preferably the natural-based polymer, is guar gum, starch, collagen, dextran, pectin, alginate, chitosan, hyaluronic acid, gellan, polypeptide and cellulose. Optionally, the natural-based polymer, preferably cellulose, can be further processed, for example by chemical derivation such as the formation of esters and ethers or pharmaceutically acceptable salts.

Guar gum, also called guaran, is a galactomannan extracted from guar beans that has thickening and stabilizing properties useful in food, feed, and industrial applications.

Starch is a polysaccharide containing a large number of $\alpha$-D-glucose units which are joined by glycosidic bonds. Starch can be produced by most green plants and used as energy storage. Starch is regarded as insoluble in cold water (23°C) but is able to exothermically swell up by physically binding a morefold of water compared to its own weight.

Collagen can be considered the main structural protein in the extracellular matrix of a body's various connective tissues. As the main component of connective tissue, it is the most abundant protein in mammals. 25% to 35% of a mammalian body's protein content is collagen. Amino acids are bound together to form a triple helix of elongated fibril known as a collagen helix. The collagen helix is mostly found in connective tissue such as cartilage, bones, tendons, ligaments, and skin.

Dextran is a complex branched polysaccharide containing glucose units only, wherein the chains have different number average molecular weights from 10.000 to 50.000.000 Daltons determined by gel permeation chromatography. The straight chain consists of glucose molecules being bonded by $\alpha$-1,6 glycosidic linkages, while branches begin from $\alpha$-1,3 linkages. Brought into contact with water, dextran forms highly viscous liquids (gels).

Pectin is a vegetable structural polysaccharide obtainable from plants such as citrus fruit plants. Pectin is a polysaccharide (more exactly a polyuronide) which mainly contains D-galacturonic acid units being $\alpha$-1,6 glycosidicly joined. All, just a part or none of the carboxy residues of the galacturonic acid in pectin can be present in an esterified form, for example as methyl or ethyl ester.

Alginate is a polysaccharide containing homopolymeric blocks of (1-4)-linked $\beta$-D-mannuronate and $\alpha$-L-guluronate (G) residues, respectively, covalently linked together in different sequences or blocks. Alginate can be present in the form of its salts, such as alkaline metal or earth alkaline metal salts, or in esterified form, such as in form of an alkyl ester, preferably methyl ester. Alginate is able to quickly absorb water to form a hydrogel. Thus, alginate can be used as gelling agent.

Chitosan is a linear biopolymer (polysaccharide) containing randomly distributed $\beta$-(1,4)-linked organic units (deacetylated unit) and N-acetyl-D-glucosamine units, wherein the polymeric chain preferably contains more D-glucosamine units than N-acetyl-D-glucosamine units. Chitosan can be obtained from chitin by deacetylation either enzymatically or via an alkaline compound such as sodium hydroxide.

Hyaluronic acid is an important component of connective tissue. Hyaluronic acid is a polymer which contains a disaccharide as repeating unit, wherein the chain comprises 250 to 50.000 of the disaccharide units. Said disaccharide comprises D-glucuronic acid and N-acetyl-D glucosamine being bound via a glycosidic $\beta$-(1,3) linkage. Hyaluronic acid can be present in the form of its salts, such as alkaline metal or earth alkaline metal salts, in particular in form of the sodium or potassium salt.

Gellan (also referred to as gellan gum) is anionic polysaccharide obtained via fermentation of carbon hydrates by a bacterium called *Pseudomonas elodea.* Gellan is a polymer containing a tetrasaccharide as repeating unit, wherein the chain comprises 250 to 50.000 of the tetrasaccharide units. Said tetrasaccharide units comprise two residues of D-glucose, one residue of L-rhamnose and a residue of D-glucuronic acid in the following order: D-glucose- D-glucuronic acid- D-glucose- L-rhamnose. A part of the D-glucose residues can be esterified with acetic acid and/or glycerol acid. Further, the D-glucuronic acid residues can be present in the form of a salt, preferably in form of the potassium, sodium, magnesium and calcium salt.

A polypeptide is a chain of amino acids linked together, wherein a single polypeptide chain might make up the entire primary structure of a simple protein. More complex proteins are formed when two or more polypeptides are linked together. Polypeptides can be naturally occurring or obtained via peptide synthesis for example via the Merrifield Synthesis.

Cellulose is a polysaccharide containing a linear chain of several hundred to many thousands of $\beta(1\rightarrow4)$-linked D-glucose units. Within the present application cellulose can be further processed, for example to obtain cellulose ethers and cellulose esters as well as their salts. Examples of cellulose ethers are hydroxyalkyl cellulose, in particular hydroxy $C_{1-6}$-alkyl cellulose, such as hydroxymethyl cellulose, hydroxyethyl cellulose, hydroxypropyl cellulose, hydroxyisopropyl cellulose and hydroxybutyl cellulose, preferably hydroxymethyl cellulose and/or hydroxyethyl cellulose. Examples of cellulose esters are carboxyalkyl cellulose, in particular carboxy $C_{1-6}$-alkyl cellulose such as carboxymethyl cellulose, carboxy ethyl cellulose, carboxypropyl cellulose, carboxybutyl cellulose and/or their salts. The number average molecular weight of cellulose and/or its derivates is 1.000 g/mol to 250.000 g/mol, preferably 5.000 g/mol to 175.000 g/mol, in particular 10.000 g/mol to 100.000 g/mol, determined with gel permeations chromatography.

Polymer (ii) is preferably a non-ionic (neutral) or cationic polymer. More preferably, polymer (ii) is a natural based, non-ionic (neutral) or cationic polymer. Examples are guar gum, starch, collagen, dextran, pectin, chitosan, gellan, polypeptide and cellulose and further processed cellulose, preferably guar gum, starch, dextran, chitosan, gellan and cellulose and further processed cellulose and mixtures thereof. Particularly preferred are guar gum, cellulose, further processed cellulose and mixtures thereof, especially hydroxy ethyl cellulose as further processed cellulose, more especially cationic hydroxy ethyl cellulose. It turned out that, when using a non-ionic or cationic polymer, the corresponding composition exhibits an advantageously high concentration of PGDF-AB in whole blood, while when using anionic polymers such as alginate, a reduced concentration of PGDF-AB in whole blood is obtained. Without being bound to any theory this may be due to trapping or bonding of said PGDF-AB in or to an anionic polymer such as alginate.

In a preferred embodiment, polymer (ii) is in the form of a hydrogel. A hydrogel can be referred to a biphasic material, a hydrogel matrix of a solid material and at least 10% by weight or volume of a fluid, preferably water. The hydrogel matrix is a substantially water-insoluble three-dimensional network of a polymer. Thus, polymer (ii) can be regarded as hydrogel matrix. Said hydrogel matrix absorbs the above-mentioned amounts of fluid(s), preferably water to form a hydrogel.

In a preferred embodiment of the invention, the content of polymer (ii) in the hydrogel is 1.0 to 15% (w/v), more preferably 1.5 to 12.5% (w/), in particular 2.0 to 10.0 (w/v).

In a particular preferred embodiment, when polymer (ii) is guar gum, the content of guar gum in the hydrogel is about 2.5% (w/v).

In a particular preferred embodiment, when polymer (ii) is hydroxyethyl cellulose, in particular cationic hydroxyethyl cellulose, the content of hydroxyethyl cellulose, in particular cationic hydroxyethyl cellulose in the hydrogel is about 8.0% (w/v).

In a preferred embodiment, the hydrogel contains at least 20 wt.%, preferably at least 30 wt.%, more preferably at least 40 wt.%, in particular at least 50 wt.% of water, wherein the hydrogel preferably contains at most 99 wt% of water.

In line with the present invention the amount of water contained in the hydrogel should be verified via DIN EN 14079, wherein the amount of water is calculated as follows:

$$W_w = \frac{W_g - W_t}{W_g} \bullet 100\% \qquad (1)$$

wherein

$W_w$ = weight of water in %, based on the total weight of the hydrogel,

$W_g$ = weight of the hydrogel

Wt = weight of the "dry component" of the hydrogel (corresponding to natural the hydrogel matrix).

In an embodiment, the hydrogel can preferably comprise an inorganic salt. The inorganic salt comprises preferably an inorganic halide, in particular chloride. In a particularly preferred embodiment, the inorganic salt is sodium chloride. The inorganic salt can preferably be present in the hydrogel in a concentration of 50 to 250 mM, more preferably in a concentration of 100 mM to 200 mM, even more preferably in a concentration of 125 to 175 mM, in particular in a concentration of about 140 mM based on the weight of the hydrogel.

Preferably the inorganic salt can be present in a concentration of about 120 mM, 125 mM, about 130 mM, about 135 mM, about 140 mM, about 145 mM, about 150 mM, about 155 mM, about 160 mM, about 165 mM, about 170 mM or about 175 mM based on the weight of the hydrogel.

In a preferred embodiment of the invention, blood (i) and hydrogel are present in a ratio (v/v) of 3:1 to 1:3, preferably, 2.5: 1 to 1:2.5, more preferably 2:1 to 1:2, even more preferably 1.5:1 to 1:1.5, in particular about 1:1.

Preferably, blood (i) and hydrogel are present in a ratio (v/v) of about 2.0:1, about 1.9:1, about 1.8:1, about 1.7:1, about 1.6:1, about 1.5:1, about 1.4:1, about 1.3:1, about 1.2:1, about, 1.1:1, about 1:1, about 1:1.1, about 1:1.2, about 1:1.3, about 1:1.4, about 1: 1.5, about 1:1.6, about 1: 1.7, about 1:1.8, about 1:1.9, or about 1:2.

The gel composition according to the invention further comprises a coagulation accelerator, wherein the coagulation accelerator is a clay mineral. A coagulation accelerator is referred to as a compound which speeds up the coagulation of blood and thus facilitates the formation of clotting.

Clay minerals can be referred to as minerals containing as main component solid hydrated aluminium silicates, in particular hydrous aluminium phyllosilicates, which may contain variable amounts of iron magnesium, alkali metals, alkaline earth metal or other cations. Non-limiting examples are halloysite ($Al_2Si_2O_5(OH)_4$), kaolin, whose main component is kaolinite ($Al_2Si_2O_5(OH)_4$), pyrophyllite ($Al_2Si_4O_{10}(OH)_2$), talc ($Mg_3Si_4O_{10}(OH)_2$), illite ($(K,H_3O)(Al,Mg,Fe)_2(Si,Al)_4O_{10}[(OH)_2,(H_2O)]$), bentonite, whose main component is montmorillonite/smectite ($(Na,Ca)_{0.33}(Al,Mg)_2Si_4O_{10}(OH)_2 \cdot n\text{-}H_2O$), chlorite ($(Mg,Fe)_3(Si,Al)_4O_{10}(OH)_2 \cdot (Mg,Fe)_3(OH)_6$), vermiculite ($(Mg,Fe,Al)_3(Al,Si)_4O_{10}(OH)_2 \cdot 4H_2O$), sepiolite ($Mg_4Si_6O_{15}(OH)_2 \cdot 6H_2O$) and palygorskite/attapulgite ($(Mg,Al)_2Si_4O_{10}(OH) \cdot 4(H_2O)$). Preferred are halloysite ($Al_2Si_2O_5(OH)_4$), kaolin and bentonite.

In a more preferred embodiment halloysite ($Al_2Si_2O_5(OH)_4$ is used as coagulation accelerator. In an alternatively more preferred embodiment kaolin is used as coagulation accelerator. Kaolin is particularly preferred.

The coagulation accelerator can preferably be present in amounts of 0.01 to 2 mg/ml whole blood, preferably 0.02 to 1 mg/ml whole blood, more preferably 0.05 to 0.1 mg/ml whole blood, in particular about 0.075 mg/ml whole blood.

In a preferred embodiment, in the present composition halloysite can be present in an amount of 0.01 to 0.5 mg/ml whole blood, preferably 0.025 to 0.35 mg/ml whole blood, more preferably 0.05 to 0.1 mg/ml whole blood, in particular about 0.075 mg/ml whole blood.

In a preferred embodiment, kaolin can be present in an amount of 0.01 to 0.5 mg/ml whole blood, preferably 0.025 to 0.35 mg/ml whole blood, more preferably 0.05 to 0.1 mg/ml whole blood, in particular about 0.075 mg/ml whole blood.

It is particularly preferred that kaolin is present in the present composition an amount about of 0.01 mg/ml whole blood, about 0.015 mg/ml whole blood, about 0.020 mg/ml whole blood, about 0.025 mg/ml whole blood, about 0.030 mg/ml whole blood, about 0.035 mg/ml whole blood, about 0.040 mg/ml whole blood, about 0.045 mg/ml whole blood, about 0.050 mg/ml whole blood, about 0.055 mg/ml whole blood, about 0.060 mg/ml whole blood, about 0.065 mg/ml whole blood, about 0.070 mg/ml whole blood, about 0.075 mg/ml whole blood, about 0.080 mg/ml whole blood, about 0.085 mg/ml whole blood, about 0.090 mg/ml whole blood, about 0.095 mg/ml whole blood, about 0.1 mg/ml whole blood.

It turned out that a kaolin is required to obtain a fast coagulation. However, using a too high an amount of kaolin leads to a reduced concentration of PGDF-AB in whole blood. Without being bound to any theory, this reduced concentration of PGDF-AB may be due to the trapping of said PGDF-AB in kaolin, if the amount of kaolin becomes too large.

In a preferred embodiment of the invention, the gel composition has a viscosity of at least 3 mPa·s at 20°C, preferably at least 10 mPa·s, more preferably at least 50 mPa·s, in particular at least 100 mPa·s. It is further preferred that the gel composition has a viscosity of at most $10^{13}$ mPa·s, preferably of at most $10^{10}$ mPa·s, more preferably of at most $10^{7}$ mPa·s, in particular of at most $10^{5}$ mPa·s. Having such a viscosity, the gel composition can be advantageously adhered to the wound and advantageously remains on the wound. The viscosity is measured as known in the art.

In a preferred embodiment of the invention, the gel composition further comprises (iv) an anti-coagulant. An anticoagulant (sometimes also referred to as blood thinner) is a compound that at least reduces or even prevents the coagulation of blood. By said slowing down the coagulation, the clotting time can be enhanced. Anticoagulants are known in the art and non-limiting examples are heparin, EDTA, citric acid, citrates, gluconate, imidosuccinate, ethylenediamine, 8-hydroxychinolin, diaceytldioxime, tartaric acid or mixtures thereof. Preferred are heparin, EDTA and citric acid, more preferred are EDTA and citric acid, in particular citric acid.

In case that the present gel composition comprises (iv) an anticoagulant, it is preferred that the gel composition further comprises (v) an agent neutralizing the effect of the anticoagulant (iv). (v) Agents neutralizing the effect of the anticoagulant are known in the art. Non-limiting examples of (v) agents neutralizing the effect of an anticoagulant are calcium halogenide or protamine, in particular calcium chloride.

In a preferred embodiment, the gel composition can be obtained by blending two sub compositions, one containing (i) and optionally (iv) anticoagulant and the other containing (ii) polymer, (iii) coagulation accelerator and optionally (v) agent neutralizing the effect of an anticoagulant.

Another aspect of the invention is a method for preparing the gel composition according to present invention comprising the steps of

(a) providing a volume of whole blood,

(b) providing a volume of polymer comprising coagulation accelerator, and

(c) mixing the whole blood from step (a) with the polymer comprising coagulation accelerator from step (b).

Generally, as far as components, amounts, ratios and the like of the method according to the invention are concerned, the same applies as described above with regard to the gel composition according to the present invention.

In step (a) a volume of (i) whole blood is provided. A volume of whole blood can be obtained by the methods known in the art. For example, whole blood, preferably autologous blood, can be obtained by blood draw via a syringe at a person to be treated. The whole blood can be processed promptly or stored for a while. In the latter case, the whole blood preferably contains (iv) an anticoagulant.

In step (b) a volume of (ii) polymer comprising (iii) coagulation accelerator is provided. It is preferred that the polymer is in the form of a hydrogel (ii) and that hydrogel and (iii) coagulation accelerator are blended to achieve a uniform distribution of the coagulation accelerator in the hydrogel. In case that (i) whole blood from step (a) contains (iv) an anticoagulant, the mixture of step (b) preferably contains (v) an agent neutralizing the effect of an anticoagulant.

Steps (a) and (b) can be carried out in this order. Alternatively step (b) can be carried out before or simultaneously to step (a).

In step (c) of the method according to the present invention, whole blood from step (a) is mixed with the polymer comprising coagulation accelerator from step (b). Mixing can be carried out by any suitable mixing device known in the art. In a preferred embodiment, blood from step (a) and polymer comprising coagulation accelerator from step (b) are combined and the resulting mixture is subjected to a mechanical treatment, such as shaking, stirring and pumped over from one container to another and back again several times. Mixing can be carried out for 5 to 600 seconds, preferably for 10 to 450 seconds, more preferably 15 to 300 seconds, in particular about 180 seconds.

In a preferred embodiment the gel composition according to the present invention is for topical administration. Contrary to systemic administration, wherein a composition is administered systemically for example via a tablet or an infusion and thus remote from the site where the therapeutic effect should be achieved, the present composition is preferably administered topically, i.e. it is administered directly to the site, preferably a wound, where the therapeutic effect should take place.

In a further aspect, the present invention concerns compositions according to the present invention for use in treatment of wounds. Examples of wounds include, but are not limited to, acute wounds, such as cuts, chronic wounds, such as decubitus, diabetic foot lesions, venous leg ulcers, arterial leg ulcers and pressure ulcers, purulent wounds, such as closed wounds resulting from an operation, infected wounds due to a local infection of the wound, traumatic wounds, such as wounds resulting from mechanical, thermic, chemical or actinic impact and ischemic wounds, which can occur due to poor blood flow. It is preferred that the composition according to the present invention is used in the treatment of a chronic wound. A wound is generally referred to as a chronic wound when the healing process cannot be finished after six to eight weeks. Chronic wounds are often due to a chronic disease and/or a chronic mechanical impact, such as friction which, for example, applies in case of decubitus.

It is preferred that the chronic wound is a wound due to diabetes or infections, pressure sores, decubitus ulcers and/or diabetic ulcers.

Further, the invention relates to a method for treating a wound, preferably a chronic wound, comprising administering to a subject in need thereof a therapeutically effective amount of a gel composition comprising (i) whole blood, (ii) a polymer, and (iii) a coagulation accelerator, wherein the coagulation is a clay mineral. For the gel composition administered in the before-mentioned method the same applies as to the gel composition as described above in the text.

In a preferred embodiment of the present invention, the present composition is administered topically in an amount of 0.01 to 3.00 $g/cm^2$, preferably 0.02 to 2.50 $g/cm^2$, preferably 0.03 to 2.00 $g/cm^2$, in particular 0.02 to 1.00 $g/cm^2$, especially about 0.1 $g/cm^2$ of the wound area. The composition can preferably be administered directly to the wound. The composition can be administered from four times a day to every three days. It is further preferred that the composition is administered once daily.

Another aspect of the invention is a kit for preparing the gel composition according to the invention, wherein the kit comprises

- one or more blood withdrawal devices with container for the withdrawn whole blood;

- one or more container(s) comprising polymer and coagulation accelerator; and

- a device for mixing whole blood and the mixture of polymer and coagulation accelerator.

Blood withdrawal devices with a container for the withdrawn whole blood are known in the art. An example is a syringe with a needle and a container.

A container(s) comprising polymer and coagulation accelerator can be any suitable container. In an embodiment, the container is a container of a syringe.

The device for mixing whole blood and the mixture of polymer and coagulation accelerator can be a device in the art. Preferred is a connecting member such that the blood can be pumped to a container comprising polymer and coagulation accelerator or *vice versa*. Subsequently, the mixture can be pumped over from one container to the other several times.

In a preferred embodiment, the kit can comprise a protective covering. Suitable protective covers such as a glove box, protective case, and wrapping are known to the skilled person. Such a protective cover should prevent the operator to come into contact with blood which might contain germs of communicable diseases.

A further aspect of the invention is a method for treating a wound comprising the steps of

(a) providing a volume of whole blood,

(b) providing a volume of polymer comprising coagulation accelerator, and

(c) mixing the whole blood from step (a) with the polymer comprising coagulation accelerator from step (b),

(d) applying the mixture from step (c) on a wound,

(e) optionally further applying a secondary dressing over the wound.

As far as steps (a) to (c) are concerned, the same consideration as with the above-described methods apply.

Step (d) is directed to applying the mixture to a wound. Further, as far the wound is concerned, the same considerations as with the wound described above apply, i.e. for example the wound to which the mixture from step (c) is to be applied is preferably a chronic wound. Applying can be carried out as known in the art. It is preferred that the mixture from step (c) is directly applied to the wound, i.e. the mixture from step (c) is not applied elsewhere, such as to a wound dressing, before brought onto the wound.

Optional step (e) is directed to further applying a wound dressing over the wound. Wound dressings to be applied over the wound are known in the art. It is preferred that the wound dressing is sterile. Further applying a wound dressing, preferably a sterile wound dressing, bears the advantage that the wound to which the mixture of step (c) is applied can be prevented from being contaminated by any pollutants and germs present in the environment.

**Exemplary section**

**Example 1: Preparation of a gel composition according to the invention**

To 10 ml of 8% hydroxyethyl cellulose in water, 0.0818 g (1.40 mmol) of sodium chloride (NaCl), 0.0184 (0.166 mmol) of calcium chloride ($CaCl_2$) and 0.75 mg of kaolin were added. The mixture was drawn into a syringe with a 60 ml container and locked (without needle) to a first access of a three-way stopcock.

Further, 10 ml of venous whole blood were drawn via an IV needle into the 60 ml container of a syringe. Subsequently the needle was removed, and the syringe was locked to a second access of the above-mentioned three-way stopcock.

The valve of the three-way stopcock was set to allow a connection of both container and the content of one container was pumped into the other container. Subsequently, the resulting composition was pumped over from one container to another 15 times to obtain the present gel composition.

**Further experiments:**

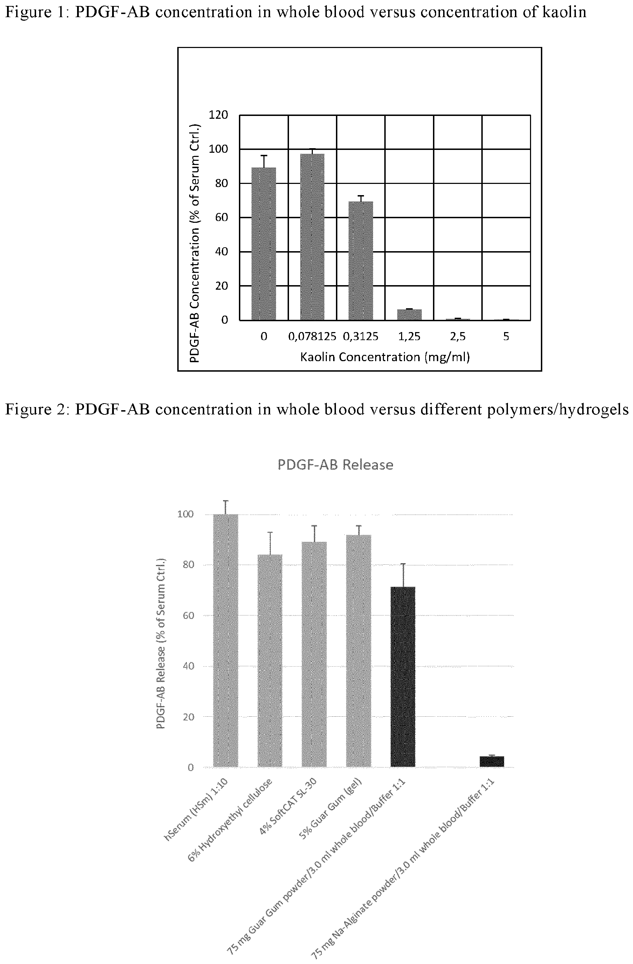

Several compositions were prepared in the same manner as Example 1, each having a different concentration of kaolin. The concentrations were as follows: 0; 0.078125 mg/ml; 0.3125 mg/ml; 1.25 mg/ml, 2.5 mg/ml and 5 mg/ml. Subsequently, the platelet-derived growth factor PDGF-AB was determined, wherein the PDGF-AB concentration in serum was used as control (100%). The results are shown in Figure 1. It is known that kaolin is required for fast coagulation. However, it can be seen that the concentration of PDGF-AB in whole blood decreases, when the concentration becomes too high. As indicated above and without being bound to any theory, this reduced concentration of PGDF-AB in whole blood may be due to trapping of said PGDF-AB in kaolin.

Several compositions were prepared in the same manner as Example 1, each having a different polymer/hydrogel. The polymer/hydrogels were as follows: 6% hydroxyethyl cellulose in water, 4% SoftCAT SL-30 (also referred to as Polyquaternium7, cationic hydroxyethyl cellulose) in water, 5% guar gum in water. Further, 75 mg guar gum powder in 3.0 ml whole blood with 1:1 buffer and 75 mg Na powder alginate in 3.0 ml whole blood with 1:1 buffer were used as compositions. Subsequently, the platelet-derived growth factor PDGF-AB was determined, wherein the PDGF-AB concentration in serum was used as control (100%). The results are shown in Figure 2. It can be seen that the concentration of PDGF-AB in whole blood is significantly higher in non-ionic and cationic polymers/hydrogels than in an anionic polymer/hydrogel such as alginate. As indicated above, the reduced concentration of PDGF-AB may be due to the trapping or bonding of said PGDF-AB in or to an anionic polymer such as alginate.

**Claims**

1. Gel composition for being provided to a wound of a patient, the composition comprising

   (i) whole blood,
   (ii) a polymer, and
   (iii) a coagulation accelerator,

   wherein the coagulation accelerator is a clay mineral.

2. Gel composition according to claim 1, wherein the polymer (ii) is a synthetic-based polymer, natural-based polymer or mixtures thereof.

3. Gel composition according to any one of claims 1 or 2, wherein the polymer (ii) is guar gum, starch, collagen, dextran, pectin, alginate, chitosan, hyaluronic acid, gellan, polypeptide, further processed cellulose or mixtures thereof.

4. Gel composition according to any one of claims 1 to 3, wherein the polymer is in form of a hydrogel.

5. Gel composition according to claim 4, wherein the content of the polymer in the hydrogel is 1 to 15% (w/v).

6. Gel composition according to claim 4 or 5, wherein whole blood (i) and hydrogel are present in a ratio (v/v) of 3:1 to 1:3.

7. Gel composition according to any one of claims 1 to 6, wherein the gel composition has a viscosity of at least 3 mPa·s at 20°C.

8. Gel composition according to any one of claims 1 to 7 further comprising (iv) anti-coagulant.

9. Gel composition according to claim 8, wherein the anticoagulant (iv) is heparin, EDTA, citric acid, citrates, gluconate, imidosuccinate, ethylendiamine, 8-hydroxychinolin, diaceytldioxime, tartaric acid or mixtures thereof.

10. Gel composition according to claim 8 or 9 further comprising (v) agent neutralizing the effect of the anticoagulant (iv).

11. Composition according to claim 10, wherein the agent (v) neutralizing the effect of the anticoagulant (iv) is a calcium halogenide or protamine.

12. Method for preparing a composition according to any one of claims 1 to 11 comprising

    (a) providing a volume of whole blood,
    (b) providing a volume of a polymer comprising coagulation accelerator,
    (c) mixing the volume whole blood from step (a) with the volume of polymer comprising coagulation accelerator from step (b).

13. Composition according to any one of claims 1 to 11 for use in the treatment of a chronic wound.

14. Kit for preparing a composition according to any one of claims 1 to 11 comprising

    - one or more blood withdrawal devices with container for the withdrawn whole blood
    - one or more container(s) comprising polymer and coagulation accelerator; and
    - device for mixing whole blood and the mixture of polymer and procoagulant.

15. Method for treating a wound comprising the steps of

    (a) providing a volume of whole blood,
    (b) providing a volume of a polymer comprising coagulation accelerator,
    (c) mixing the volume whole blood from step (a) with the volume of polymer comprising coagulation accelerator from step (b),
    (d) applying the mixture from step (b) on the wound,
    (e) optionally further applying a secondary dressing over the wound,

    wherein the coagulation accelerator is a clay mineral.

Figure 1: PDGF-AB concentration in whole blood versus concentration of kaolin

Figure 2: PDGF-AB concentration in whole blood versus different polymers/hydrogels

Europäisches
Patentamt

European
Patent Office

Office européen
des brevets

# EUROPEAN SEARCH REPORT

**Application Number**

EP 24 19 1857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/319476 A1 (WARD KEVIN R [US] ET AL) 25 December 2008 (2008-12-25) <br> * page 1, paragraph 13 - page 2, paragraph 14 * <br> * page 2, paragraph 27-29 * <br> * page 3, paragraph 34 * <br> * page 5, paragraph 60-61 * <br> * page 5, paragraph 71 - page 6, paragraph 83 * <br> * claims * | 1-15 | INV. <br> A61L26/00 <br> A61K35/14 <br> A61P17/02 |
| A | Kushnir Igal ET AL: "Efficacy and Safety of a Novel Autologous Wound Matrix in the Management of Complicated, Chronic Wounds: A Pilot Study", <br> Index Wounds, <br> 1 September 2016 (2016-09-01), <br> XP055912334, <br> Retrieved from the Internet: <br> URL:https://www.hmpgloballearningnetwork.com/site/wounds/article/efficacy-and-safety-novel-autologous-wound-matrix-management-complicated-chronic-wounds <br> [retrieved on 2022-04-13] <br> * the whole document * | 1-15 | |
| A | WO 2023/144818 A1 (REDDRESS LTD [IL]) 3 August 2023 (2023-08-03) <br> * page 4, lines 4-10 * <br> * claims * | 1-15 | TECHNICAL FIELDS SEARCHED (IPC) <br><br> A61L <br> A61K <br> A61P |
| A | US 2012/321721 A1 (OUYANG WEI [CA] ET AL) 20 December 2012 (2012-12-20) <br> * page 2, paragraph 15-16 * <br> * page 3, paragraph 37 * <br> * page 5, paragraph 53 * <br> * claims * | 1-15 | |

-/--

The present search report has been drawn up for all claims

| Place of search | Date of completion of the search | Examiner |
|---|---|---|
| The Hague | 7 January 2025 | Van den Bulcke, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 1857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIU TAO ET AL: "Electrospun kaolin-loaded chitosan/PEO nanofibers for rapid hemostasis and accelerated wound healing", INTERNATIONAL JOURNAL OF BIOLOGICAL MACROMOLECULES, ELSEVIER BV, NL, vol. 217, 28 July 2022 (2022-07-28), pages 998-1011, XP087165726, ISSN: 0141-8130, DOI: 10.1016/J.IJBIOMAC.2022.07.186 [retrieved on 2022-07-28] * the whole document * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 7 January 2025 | Van den Bulcke, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ........................................................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 19 1857

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2025

| Patent document cited in search report | Publication date | Patent family member(s) | Publication date |
|---|---|---|---|
| US 2008319476 A1 | 25-12-2008 | US 2008319476 A1 | 25-12-2008 |
| | | WO 2008153714 A1 | 18-12-2008 |
| WO 2023144818 A1 | 03-08-2023 | AU 2023214201 A1 | 08-08-2024 |
| | | CN 118613239 A | 06-09-2024 |
| | | EP 4447889 A1 | 23-10-2024 |
| | | IL 290122 A | 01-03-2022 |
| | | KR 20240144143 A | 02-10-2024 |
| | | US 2024374636 A1 | 14-11-2024 |
| | | WO 2023144818 A1 | 03-08-2023 |
| US 2012321721 A1 | 20-12-2012 | CA 2814783 A1 | 26-05-2011 |
| | | DK 2501754 T3 | 03-09-2018 |
| | | EP 2501754 A1 | 26-09-2012 |
| | | ES 2688977 T3 | 07-11-2018 |
| | | US 2012321721 A1 | 20-12-2012 |
| | | US 2017333477 A1 | 23-11-2017 |
| | | WO 2011060544 A1 | 26-05-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010000451 A
- WO 2011141454 A
- EP 1294414 B1
- US 9180142 B2

**Non-patent literature cited in the description**

- **SHULA VK et al.** Evaluation of pH measurement as a method of wound assessment. *J. Wound Care*, 2007, vol. 16 (7), 291-294